Europäisches Patentamt

(19) European Patent Office

Office européen des brevets

(11) Publication number : **0 482 847 A1**

(12) **EUROPEAN PATENT APPLICATION**

(21) Application number : **91309671.5**

(22) Date of filing : **18.10.91**

(51) Int. Cl.$^5$ : **A61F 9/00**

(30) Priority : **26.10.90 US 603372**
**05.04.91 US 681686**

(43) Date of publication of application :
**29.04.92 Bulletin 92/18**

(84) Designated Contracting States :
**AT BE CH DE DK ES FR GB GR IT LI LU NL SE**

(71) Applicant : **ALCON SURGICAL, INC.,**
**6201 South Freeway**
**Forth Worth, Texas 76134-2099 (US)**

(72) Inventor : **Beuchat, Charles E.**
**6 Westport**
**Irvine, California 92720 (US)**
Inventor : **Ureche, Alexander**
**27402 Via Caudaloso**
**Mission Viejo, California 92692 (US)**

(74) Representative : **Woodcraft, David Charles et al**
**BROOKES & MARTIN High Holborn House**
**52/54 High Holborn**
**London, WC1V 6SE (GB)**

(54) **Method and apparatus for selectively removing body tissue.**

(57)   The disclosure relates to a method and apparatus for selectively removing body tissue through application of ultrasonic energy being applied to improved surgical tips.

FIG. 1

EP 0 482 847 A1

Jouve, 18, rue Saint-Denis, 75001 PARIS

This application is a continuation-in-part application of United States Patent Application Serial Number 07/603,372, filed October 26, 1990.

## Background of the Invention

The present invention relates generally to surgical tip assemblies, surgical apparatus and method for removing body tissue. More particularly, it relates to surgical tip assemblies, surgical apparatus and methods for ultrasonically removing preselected portions of body tissue from a surgical site.

A wide variety of ultrasonic surgical tip assemblies exist for use in combination with ultrasonic surgical apparatus for use in the field of microsurgery. In the field of ophthalmic surgery, these surgical tips and apparatus have been used extensively. Representative examples of such surgical tip assemblies and surgical apparatus are described in U.S. Patent Nos. 3,589,363; 4,223,676; 4,246,902; 4,493,694; and 4,922,902. In practice, these ultrasonically vibrated surgical devices are able to precisely fragment and remove unwanted tissue. Basically with such apparatus, the ultrasonically vibrating surgical tip assembly is comprised of a hollow metallic tube which is caused to ultrasonically vibrate by an ultrasonic transducer housed within the surgical handpiece. The surgical tip assembly is operative to fragment the tissue it contacts. For removing the fragmented tissue from the surgical site, the hollow surgical tip assembly is connected to an appropriate source of vacuum which is generally contained in a separate console. The fragmented tissue and other material are aspirated through the tip and ultimately to a collection container associated with the console. As is frequently the case in the use of an ultrasonic aspirator handpiece, a source of irrigation fluid is provided. The irrigation fluid not only cools the tip, but facilitates removal of the fragmented tissue and in the case of ophthalmic surgery, supplies pressure to the interior of the eye. Thus, prior art surgical tips facilitate tissue cutting or fragmentation due to the ultrasonic impacts delivered to the tissue by the tip.

With ultrasonic fragmentation in ophthalmic surgery, minimization of cavitation generated bubbles in the irrigation fluid surrounding the tip is sought. This is because such bubbles tend to visually obscure the surgical view during cataract removal procedures. Accordingly, surgical tip surfaces generating cavitation bubbles are not desired. The known prior art relating to surgical tips has, in general, sought to minimize the production of cavitation bubbles in the irrigation fluid because they, among other reasons, have a tendency to interfere visually with the surgical procedure.

On the other hand, for example, a different approach is discussed in U.S. Patent No. 4,922,902 which describes enhancing tissue removal by effecting intracellular cavitation. Towards that end, it is suggested that intracellular cavitation is enhanced by increasing vibrational amplitude or acceleration applied to the tissue to be removed. Additionally, it is suggested that in addition to lowering the frequency of vibration, for example to 10-20 Hz, the cavitation rate is further improved by increasing available tip velocity. This approach, however, is concerned with lowering vibiational frequency to achieve intracellular cavitation.

While there are a wide variety of known surgical tip configurations which are used in phacoemulsification techniques, none is known to provide extracellular collapsible micro-cavitation bubbles adjacent the tissue to be removed for enhancing the destruction of such tissue.

None of the prior art heretofore has shown or suggested modifying surgical tip structures for establishing such cavitation in the fluid surrounding the tissue to be removed for purposes of emulsifying such tissue. Neither has the prior art shown cavitation for sculpting tissue, such as sculpting the cornea.

## Summary of the Invention

According to the present invention, there are provided improved ultrasonic surgical tip assemblies for use in conjunction with an ultrasonic aspiration apparatus and an improved method for removing biological material.

The surgical tip assembly includes a surgical body having a cutting surface adjacent a distal end thereof. The body has at least a cavitation generating surface. As used herein the term "cavitation generating surface" means a surface capable of producing collapsible cavitation bubbles in a region adjacent the tissue to be removed in response to ultrasonic vibrations, said bubbles collapse releasing energy sufficient for destroying or emulsifying the adjacent tissue.

In an illustrated embodiment, the surgical body includes an aspiration passage having an aspiration port adjacent the cutting surface. In this illustrated embodiment, the cutting edge is adapted to engage tissue to be removed.

In another illustrated embodiment, the cavitation generating surface is formed by a blunt surface oriented at an angle to the direction of vibration.

In another illustrated embodiment, the cutting edge is provided or positioned at a first predetermined angle relative to a longitudinal axis of the surgical body; and the cavitation generating surface is formed along a second predetermined angle relative to the first predetermined angle.

In another illustrated embodiment, a second collapsible cavitation generating surface is positioned adjacent the first cavitation generating surface.

Another aspect of the present invention relates to an ultrasonic aspiration apparatus comprising housing means, transducer means mounted in the housing means and operable for converting electrical energy supplied thereto into mechanical ultrasonic vibrational energy which is useful for disintegrating body tissue at an operative site. A vibrational coupling means is provided which transmits the vibrational energy of the transducer means to an operative tip assembly. The operative tip assembly includes a surgical body having a cutting surface adjacent a distal end thereof. The body has at least a cavitation generating surface.

In another aspect of this invention, the cavitation can be used for sculpting the cornea.

In another aspect of this invention, the cavitation can be used in removing cataract lenses.

In an illustrated embodiment of the apparatus, the surgical body includes an aspiration passage having an aspiration port adjacent the cutting surface. In this illustrated embodiment, the cutting edge is adapted to engage tissue to be removed.

In another illustrated embodiment of the apparatus, the cavitation generating surface is formed by a blunt surface oriented at an angle to the direction of vibration.

In another illustrated embodiment of the apparatus, the cutting edge is provided or positioned at a first predetermined angle relative to a longitudinal axis of the surgical body; and the blunt surface provided is formed along a second predetermined angle relative to the first predetermined angle.

In another illustrated embodiment of the apparatus, a second collapsible cavitation generating surface is positioned adjacent the first cavitation generating surface.

A still further aspect of the present invention is a method for removing biological material from a surgical site. The method includes the step of providing an ultrasonically vibratable surgical tip assembly for removing body tissue. The tip assembly includes at least a cavitation surface capable of producing collapsible micro-cavitation bubbles. Also included is step of ultrasonically vibrating the cavitation producing surface so as to produce the cavitation bubbles which collapse in fluid immediately adjacent the tissues to be removed so that upon collapsing of the bubbles in the region of such tissues, energy is released so as to thereby destroy the structure of the tissue. The destroyed or emulsified tissue is aspirated from the surgical site.

A still further aspect of this invention is utilization of the cavitation to effect sculpting of body tissue, such as the cornea.

In an illustrated embodiment, the fluid in which the cavitation bubbles are generated is irrigation fluid supplied to the surgical site, and which is also removed therefrom by aspiration.

In another illustrated embodiment, the method comprises the steps of vibrating a hollow cutting tool that has a longitudinal portion along a longitudinal axis and a bent tip portion having a cutting edge at the distal end thereof. The bent tip portion is formed at an oblique angle relative to the longitudinal axis. The process includes the step of providing a blunt cavitation generating surface immediately adjacent and at an oblique angle to the bent tip portion so that during vibration of the blunt tip portion, extracellular micro-cavitation bubbles are formed. These bubbles, upon collapse, will release energy to augment the cutting action of the cutting edge. The method includes aspirating the cut and/or emulsified tissue from the surgical site.

Among the other objects and features of the present invention are the provisions for an improved method, ultrasonic surgical apparatus and surgical tip assembly for use in increasing the removal rate of biological material from a surgical site; the provisions for an improved method, ultrasonic surgical apparatus and surgical tip assembly for providing extracellular tissue destroying cavitation bubbles; the provisions of an improved method, surgical apparatus, and surgical tip assembly which utilizes cavitation generating surfaces capable of producing collapsible micro-cavitation bubbles for emulsifying the tissue adjacent the bubbles; the provision of an improved method, surgical apparatus and tip assembly which generates the micro-cavitation bubbles for use in sculpting body tissue, such as a cornea; the provisions for an improved method, surgical apparatus and surgical tip assembly for providing a plurality of cavitation generating surfaces adjacent a distal end of the surgical tip; the provisions for an improved method, surgical apparatus and surgical tip assembly of the last noted type wherein a cutting edge is formed adjacent the cavitation generating surface; the provisions for an improved method, surgical apparatus and surgical tip assembly of the last noted types which further includes an aspiration port adjacent the cutting edge and cavitation generating surface; the provision for an improved method, surgical apparatus and tip assembly in which the fluid selected for generating the cavitation bubbles enhances formation of such bubbles; the provisions for an improved method, surgical apparatus and surgical tip assembly for significantly lowering the force used by a physician in performing an operation; the provisions for an improved method, surgical apparatus and surgical tip assembly for significantly reducing power needed for cutting; and, the provisions for an improved method, surgical apparatus and surgical tip assembly which enhances tissue removal efficiency.

Still other objects and further scope of applicability of the present invention will become apparent from the detailed description to follow when taken in conjunction with the accompanying drawings in which like parts are designated by like reference numerals throughout the several views.

Brief Description of Drawings

Fig. 1 is a schematic view of an ultrasonic apparatus according to the present invention;

Fig. 2 is a schematic view partly in cross-section, illustrating details of certain components of the present invention;

Fig. 3 is an enlarged fragmented view of an improved high-energy tip according to one aspect of the present invention;

Fig. 3A is an end elevational view depicting a distal end of the tip shown in Fig. 3;

Fig. 4 is another preferred embodiment of an improved tip according to the present invention;

Fig. 5 is another preferred embodiment of an improved tip according to the present invention;

Fig. 6 represents still another preferred embodiment of the present invention;

Fig. 6A is an enlarged fragmented portion of a tip as shown in Fig. 6;

Fig. 7 is still another preferred embodiment of the present invention;

Fig. 8 is still another preferred embodiment of the present invention;

Figs. 9-10 respectively represent still other preferred embodiments of the present invention;

Fig. 11 is a diagrammatic view of still another preferred embodiment of the present invention;

Fig. 12 is still another preferred embodiment of the high-energy tip of the present invention; and

Fig. 13 shows a further embodiment of the high-energy tip depicted in Fig. 12.

Detailed Description

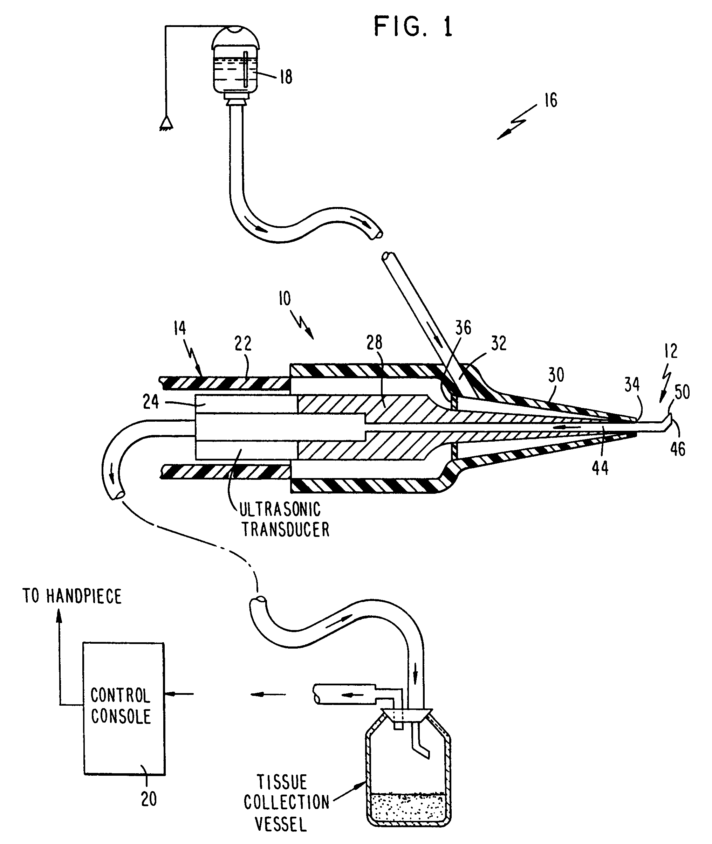

Reference is made to Figs. 1-3A for illustrating one preferred embodiment of an improved ultrasonic aspirator apparatus 10 made according to the present invention. The aspirator apparatus 10 includes an improved surgical tip assembly 12 and an ultrasonic vibratable surgical handpiece 14. A wide variety of ultrasonic aspirator apparatus are known in the art and one is described in U.S. Patent 3,693,613. This patent is incorporated herein by reference so as to provide a more detailed description of basic components and operations of the surgical ultrasonic aspirator 10 used in this embodiment. It is contemplated that irrigating fluid may not be supplied to the operative site in certain situations. Accordingly, the irrigating system described for use in the last noted patent need not be used. In addition to the aspirator apparatus, there is also provided a surgical system 16 to be used in conjunction with the handpiece 14. Included in the surgical system 16 is a source 18 of irrigation fluid and a control console 20 whose operation controls many functions of the handpiece and surgical system. Since such a control console is known, a detailed description thereof is omitted. For example, a control console usable with the apparatus 10 is commercially available from Alcon Surgical, Inc. of Fort Worth, Texas. Other types are contemplated.

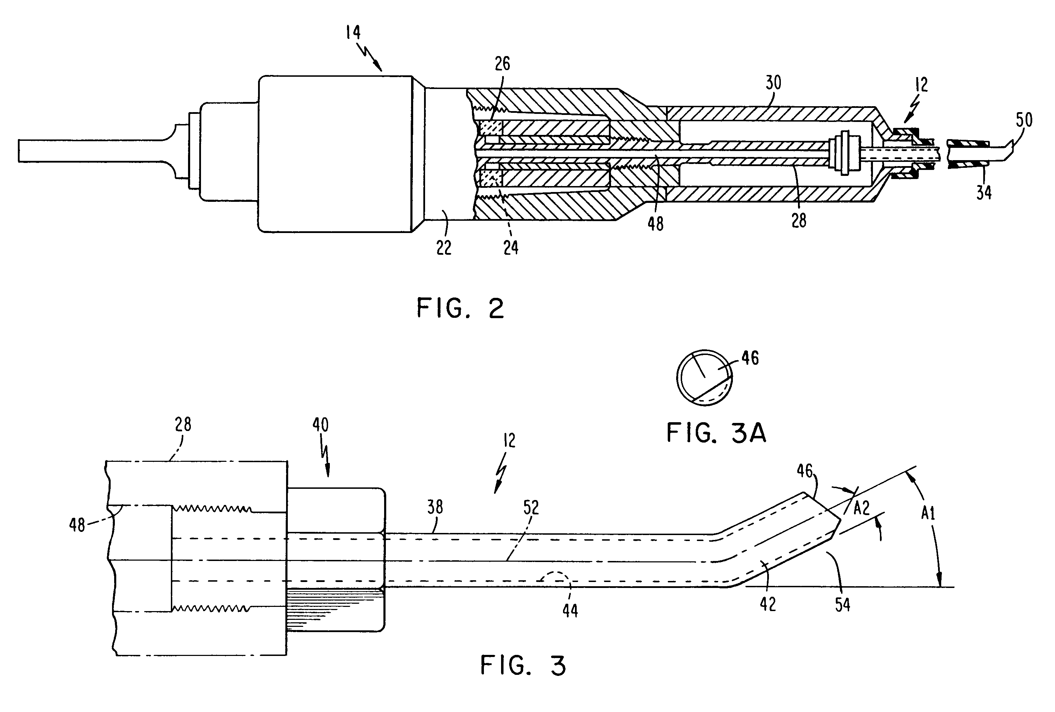

Reference is made to Figs. 1 and 2 for illustrating the ultrasonic aspirator handpiece 14. Essentially, it includes a housing assembly 22 which supports and houses an ultrasonic transducer assembly 24. The ultrasonic transducer assembly 24 can be made from several which are known in the art. For example, electrodynamic magnetostrictive or piezoelectric types can be used. If a magnetostrictive type is used, some transducer assembly cooling might be needed. However, in this particular embodiment, the transducer 24 assembly is comprised of a pair of piezo-ceramic crystals 26. The transducer crystals 26 are operable in response to high frequency electrical energy supplied from a suitable power source (not shown) in the control console 20 to produce high frequency ultrasonic mechanical vibrations in the surgical tip assembly 12. The ultrasonic transducer assembly 24 produces ultrasonic vibrations in a range of about 20-100 KHz with 40 KHz being preferred for ophthalmic surgery. The ultrasonic vibrations are transmitted to the surgical tip assembly 12 by a known connecting assembly or ultrasonic horn assembly generally designated by reference numeral 28. Basically, the ultrasonic transducer assembly 24 generates longitudinal ultrasonic vibrational waves. Elliptical vibrational wave patterns of the tip assembly are also contemplated.

With continued reference to Figs. 1 and 2, the surgical tip assembly 12 is surrounded by an irrigation sleeve 30 which is capable of providing an irrigation passage for irrigation fluid from the source 18 to the operative site. A proximal end of the sleeve 30 is coupled to one end of the handpiece housing assembly 22 and includes an inlet 32 (Fig. 1) connectable to tubing from the irrigation fluid source 18. The opposite or distal end of the sleeve provides an annular outlet opening 34 coaxial with the tip assembly 12 for delivering the irrigation fluid to the operative site. The sleeve 30 terminates short of the tip 12. Interposed between the sleeve 30 and the ultrasonic horn 28 is a suitable sealing mechanism 36 which serves to confine the irrigation fluid within the sleeve.

Additional reference is made to Figs. 3 and 3A for purposes of illustrating a first preferred embodiment of the surgical tip assembly 12. Included is an elongated tubular tip member 38 being coupled at a proximal end 40 thereof, with a suitable leading end of the ultrasonic horn 28. The tip assembly 12 has a cutting tip portion 42 at a distal end thereof and an aspiration passage 44 having an aspiration port 46. A cutting surface or edge 50 is formed on the cutting tip portion 42 adjacent the aspiration port 46 and is arranged to engage and cut through the body tissue (e.g. cataractous lenses).

The aspiration passage 44 and aspiration port 46 are in fluid communication with an aspiration passage 48 extending through the horn 28 and the handpiece 14 and lead to a source of aspiration, such as a vacuum pump (not shown) in the control console 20. Tubing is used to connect the handpiece 14 to the console 20. The control of aspiration at the operative can be regulated by a suitable control mechanism, for example a footswitch not shown and not forming part of the present invention which controls the console 20. The aspirated contents are transported ultimately to a vacuum collection vessel.

The operative tip assembly 12, as will be described, is effective to establish collapsible micro-cavitation bubbles in the fluid immediately adjacent a cutting edge 50 and the tissue to be removed. By providing for such cavitation, the destruction of such tissue is enhanced significantly. It has been determined that when the micro-cavitation bubbles collapse, they release sufficient energy which is transmitted to the tissue contacted so as to emulsify such tissue. Accordingly, this destruction of tissue is in addition to the cutting action which is provided independently by the cutting edge 50. Hence, the destruction of the unwanted body tissue is increased significantly. Accordingly, less power need be used because of the higher efficiency of the ultrasonic vibration.

While the usual operating procedure contemplated involves engagement by the cutting edge 50, tissue can be destroyed or emulsified by merely forming the collapsible micro-cavitation bubbles immediately adjacent such tissue. The micro-cavitation bubbles which are contemplated for use by this invention result from extra-cellular cavitation which is generated in the irrigation and/or body fluid surrounding the tissue to be removed.

With continued reference to Figs. 3 and 3A, the cutting edge 50 is facing upwardly relative to a longitudinal axis 52 of the tubular portion 38. For generating the extra-cellular micro-cavitation bubbles, there is provided a cavitation generating surface 54, which in this embodiment has as blunt configuration. Thus, provision is made for an angular stroke by the blunt surface 54. These vibrations of the tip occur longitudinally, but elliptical vibrations thereof are also envisioned. Accordingly, cavitation is generated because of the blunt surface 54 being at an angle to the path of tip displacement. By having the blunt surface immediately adjacent the sharpened cutting edge 50, it has been determined that tissue emulsification is even more increased relative to being at other locations.

The blunt cavitation generating surface 54 is formed by flattening at least a small portion of the bent distal cutting tip portion 42. The cutting tip portion 42 is bent upwardly at a first predetermined angle relative to the longitudinal axis 52. In this embodiment, the first predetermined angle A1 is, for example, 30°. For purposes of illustration, this bend angle A1 can vary from about 10° to 90°. The blunt bubble generating surface 54 is formed at a second predetermined angle A2 relative to the first predetermined bend angle of the tip portion 42. In this particular embodiment, the blunt surface 54 can be formed at the angle A2 relative to the first predetermined bend angle A1 in a range from about 10° to 90°, for example 30° is used in this embodiment. The blunt surface 54 is instrumental in providing the collapsible micro-cavitation bubbles in the fluid immediately adjacent the cutting edge 50. By having the blunt surface 54 immediately adjacent the cutting edge 50, such arrangement increases efficiency to facilitate greatly the reduction in power necessary to effect cutting of the tissue.

In this particular embodiment, the surgical tip assembly 12 has an outer diameter of 1 mm and the bent cutting tip portion 42 is 1 mm in length as measured from the bend radius to the cutting edge 50. The blunt surface 54 is about 0.3 mm in length. It will be appreciated that other dimensions and angles for the blunt surface 54 are envisioned so long as they are effective to achieve the desired collapsible micro-cavitation bubbles immediately adjacent the tissue to be removed. Thus, the power supplied by the transducer assembly 24 can be reduced. The stroke of the cutting tip portion 42 should be in a range of from about 0.3 mils to 6.0 mils, with 1.5 mils to 1.8 mils being preferred so as to effect the desired production of collapsible micro-cavitation bubbles. It has been determined that the desired micro-cavitation bubbles are generated by the cutting tip portion 42 reaching a threshold velocity. The threshold velocity is that velocity necessary for propagating visible cavitation bubbles. For purposes of illustration, in an ophthalmic situation, wherein the frequency is 40 KHz and the stroke is 1.5 mils, the threshold velocity is in the order of about 60 in. per second. Such is useful in cataract removal. Other operating frequencies and tip stroke lengths are envisioned consistent with the end of providing visible micro-cavitation bubbles collapsible generally immediately adjacent the blunt surface 54. The tip assembly 12 is preferably made of a low acoustic impedance material, such as titanium, to facilitate the formation of the micro-cavitation bubbles.

The micro-cavitation bubbles are bubbles which are generally smaller than the cavitation bubbles generally encountered in ophthalmic surgery. The term micro-cavitation bubbles, as used in the specification and claims means bubbles which are of such a size as to collapse relatively rapidly after formation in the fluid and, thereby release sufficient energy which effects the desired tissue emulsification. The micro-cavitation bubbles are generally in a size range which achieves the desired collapsing adjacent the tip soon after formation thereof. While the present invention prefers micro-cavitation bubbles, it envisions use of cavitation bubbles which collapse adjacent the tissue to be destroyed and release energy for destruction of tissue.

When the blunt surface 54 is moved through the irrigating fluid or body liquid, there will be a change in the magnitude or direction of the velocity of the liquid, thereby causing pressure changes therein. At some point on the surface of the blunt surface 54, the pressure will have a minimum value. This minimum is usually where a velocity is highest. If at any point the local velocity is so high that the pressure is reduced to the liquid's vapor pressure, the liquid will vaporize and cavitation bubbles will form. If the pressure then becomes higher, the bubbles of the vapor will contract and collapse. This action will observe a very high dynamic pressure on any adjacent solid structure. Additionally, according to the present invention, the size of the cavitation bubbles are inversely proportional to the frequency of vibrational movement. Specifically, the higher the frequency, the smaller the bubbles. According to the present invention, adding micro-cavitation bubbles at selected points of the tip will allow for more efficient use of the driving energy. Hence, not only is the tissue destroyed by the collapsing micro-cavitation bubbles, but also because of impacts of the cutting edge. It has also been analyzed that the tip 12 and the blunt surface 54 show that the stroke has a vector component perpendicular to the blunt surface which will generate localized cavitation bubbles. As a result, cutting efficiency is increased, thereby allowing a reduction in the preset cutting power by about up to 50% compared to prior operative tips without cavitation generating surfaces. Experiments have shown that cataracts can be removed at a reduction of 30-40% of the power typically associated with prior operative tips. Also, the force needed to remove tissue is reduced. This has an obvious advantage in that it is no longer necessary to push on the lens and thereby the zonules during the phacoemulsification process.

Furthermore, the present invention, by having the aspiration port away from the direction of travel, in those cases where the tip 12 is facing away from the direction of travel, gives a physician more confidence while working near the capsular bag. Another advantage of the present surgical tip is the fact that the likelihood of coring and thereby clogging of the aspiration passage is diminished significantly. As noted, the extra-cellular cavitation produces additional tissue emulsification in addition to the normal cutting action of the cutting edge. In this manner, the rate of tissue removal is increased independent of the vacuum level.

For bending the extremely thin tubular portion 38 without kinking thereof, multiple filaments of wire (not shown) are inserted into the port 46 prior to bending around a conventional radius tool. The wire filaments serve to prevent the tubular portion 38 from collapsing and are also easily removable one strand at a time upon completion of the bending.

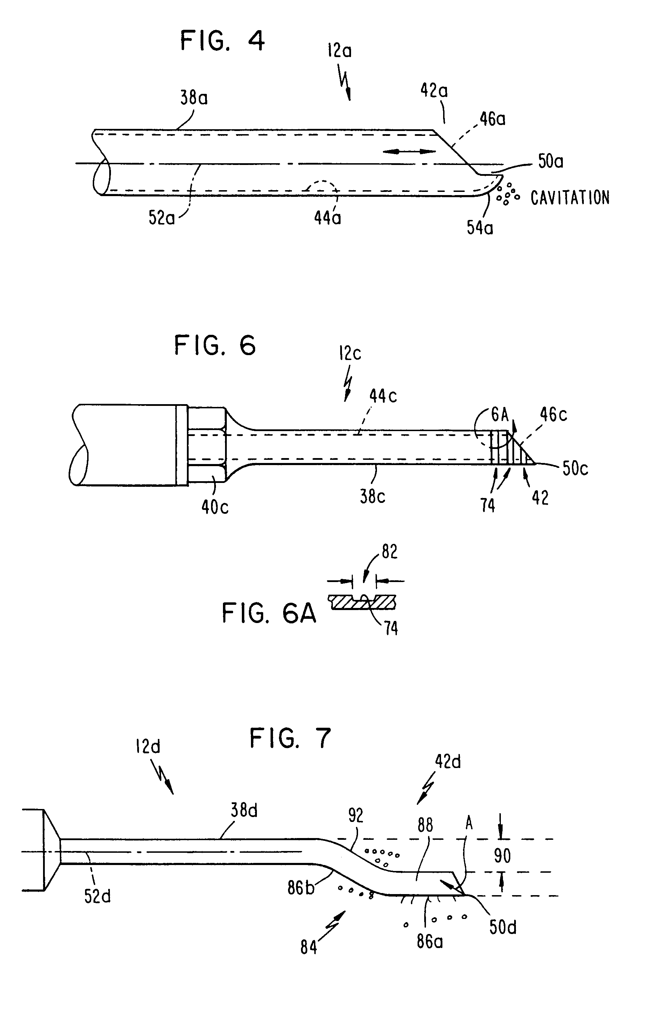

Reference is made to Fig. 4 for illustrating another preferred embodiment of the surgical tip assembly 12a. In this embodiment, there is no bent tip portion. Instead, the tip portion 42a is elongated, but it has a flattened or blunt cavitation generating surface 54a. For purposes of illustration, the surface 54a can be at an angle in a range from about 30° to 90° with 45° being preferred relative to a longitudinal axis 52a of the tip assembly 12a. The blunt surface 54a is immediately adjacent the cutting edge 50a and the aspiration port 46a. The extent of the blunt surface 54a, as shown in Fig. 4, is from about 0.3 mm. As in the previous embodiment, the blunt surface 54a is configured to propagate the micro-cavitation bubbles, which as noted quickly collapse adjacent the tissue to be destroyed. Therefore, the cavitation generating surface enhances significantly tissue emulsification.

Reference is now made to Fig. 5 for illustrating still another preferred embodiment of the present invention. In this embodiment, the surgical tip assembly 12b is not provided with an aspiration passage. Instead, the tip assembly 12b is provided with an ultrasonically driven solid surgical body or rod 60 which has a sharpened annular edge 62 and generally concave forward end 64. The emulsifying rod is generally cylindrical and is threadedly attached to a generally elongated ultrasonic horn 28b such as generally shown in Fig. 5. In this embodiment, the ultrasonic horn 28b has the same outer diameter as the ultrasonically driven rod 60. Surrounding the rod 60 and horn 28b is a tubular aspiration 66 sleeve, the distal end of the sleeve 66 terminates prior to the end of the ultrasonically driven rod 60 so that the latter protrudes an adequate distance for facilitating emulsification of the tissue T. As with the other embodiment, the cutting edge 62 need not directly contact the tissue which is to be removed because the extra-cellular cavitation produced by the concave surface 64 effects the desired tissue emulsification. An opposite end of the tubular aspiration sleeve 66 is connected to an aspiration outlet 68. The aspiration outlet 68 is connected, such as by suitable tubing (not shown), to the control console 20 housing a suitable vacuum pump which is controlled to provide the desired aspiration or negative pressure necessary to aspirate the fragmented tissue and fluids from the operative site. In this embodiment, there is provided an irrigation sleeve 70 which is generally coaxial to the aspiration sleeve 66 and supplies irrigating fluid to the operative site.

Reference is now made to Figs. 6 and 6A for illustrating a still further embodiment of the present invention. In this embodiment, the surgical tip assembly 12c is provided with a plurality of cavitation generating surfaces which are surface irregularities in the form of circumferential grooves 74 at the distal end thereof which is adjacent the cutting edge 50c. The distal tip portion 42c in this embodiment is straight. However, the surface irregularities or grooves 74 are formed, preferably, within 1 mm (0.040 in.) of the cutting edge 50c. The grooves 74 have width 82 of about 0.006 in. and have a depth which can range from .002 to .003 in. In this embodiment, the micro bubbles are formed by the grooves 74 since the latter define low pressure areas when the ultrasonic tip assembly 12c is ultrasonically vibrated. While the grooves 74 are preferred for purposes of forming low pressure depressions in the surface of the tip, it will be realized that other configured depressions may be formed so long as they provide the desired low pressure in an area or region immediately adjacent the distal end so as to form the collapsible cavitation bubbles. Although not shown, the tubular aspiration and irrigation sleeves would be used in this embodiment as with the last embodiment.

Reference is now made to Fig. 7 for purposes of disclosing another preferred embodiment of a surgical tip assembly 12d. This one has a double bend operative tip or so-called dog-leg tip 84. In this embodiment, as in the previous embodiment, the cylindrical double bend operative tip 84 is vibrated so as to effect formation of the collapsible micro-cavitation bubbles formed by this invention. The leading surface 86a is a cavitation generating surface which provides the desired collapsible micro-cavitation bubbles during the forward stroke, while the cavitation generating surface 86b generates the collapsible micro-cavitation bubbles during the return stroke. The tubular distal end portion 42d has an elongated portion 88 generally parallel to the axis 52d by an offset 90, therefrom by a suitable distance such as 0.08 in. A first bend portion 92 connects the elongated portion 88 to the tubular portion 38d. This offset dimension can vary. For example, the first bend portion 92 bends downwardly at an angle which is arranged to achieve the desired offset. The length of the straight portion 88 and the bent portion 92 can vary. The degree of angular bending and the lengths of such portions 88, 92 determine the amount of micro-cavitation bubbles assuming other discussed cavitation producing factors are constant. The degree of offset can vary so long as it yields the desired cavitation. The micro-cavitation bubbles propagated are generally transverse to the circumferential surfaces 86a and 86b. It is desired to have the micro-cavitation bubbles generated during both the forward and return strokes. Arrow A indicates the direction of stroke movement of the tip 42d caused by the offset which is effective to produce the desired cavitation.

This offset is effective in various kinds of surgery, preferably, ophthalmic surgery. In this latter regard, the tip 84 can be used for a capsulotomy as well as iris removal, not to mention cataract removal. This embodiment contemplates use of both the aspiration and irrigation sleeves although the tip 54 can have an aspiration opening adjacent the cutting edge 50d.

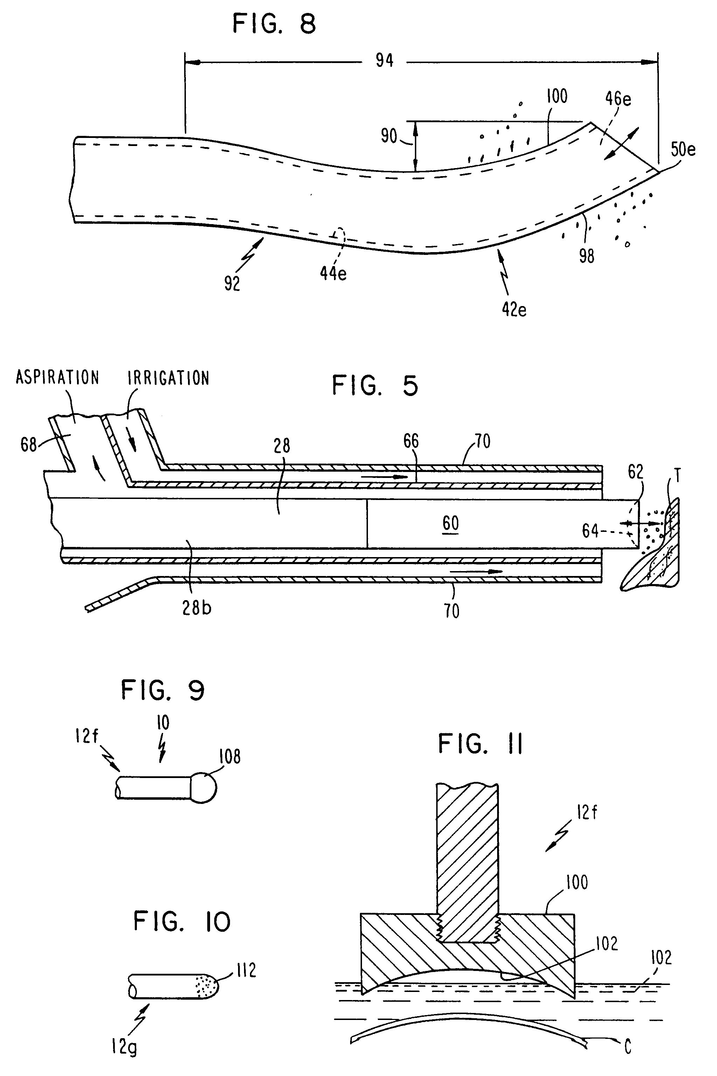

Reference is made to Fig. 8 for illustrating another embodiment of a surgical tip 92. In this particular embodiment, the distal tip end portion 42e has a curved configuration as illustrated. The length 94 of the surgical tip 92 is bout 0.68 in. and the length 94 of the curved tip portion 42e extends 0.22 in. The radius of curvature 96 of the curved tip 42e is about 0.025 in. whereas the outside diameter of the tip 12e is 0.045 in. Other dimensional configurations are contemplated. As a result of this particular configuration, micro-cavitation bubbles are produced because the movement component of the tip portion 42e is generally perpendicular to the cavitation generating surface 98. It will be noted that the cavitation generating surface 98 produces the collapsible micro-cavitation bubbles during the forward stroke, whereas the upwardly curved cavitation generating surface 100 generates the same type of micro-cavitation bubbles during the return stroke. In this embodiment, the tip 92 is depicted as including a central aspiration passage 44e and an aspiration port 46e adjacent cutting edge 50e. The tip 92 can also be solid and, of course, the aspiration and irrigation can be conducted by appropriate generally concentric sleeves (not shown).

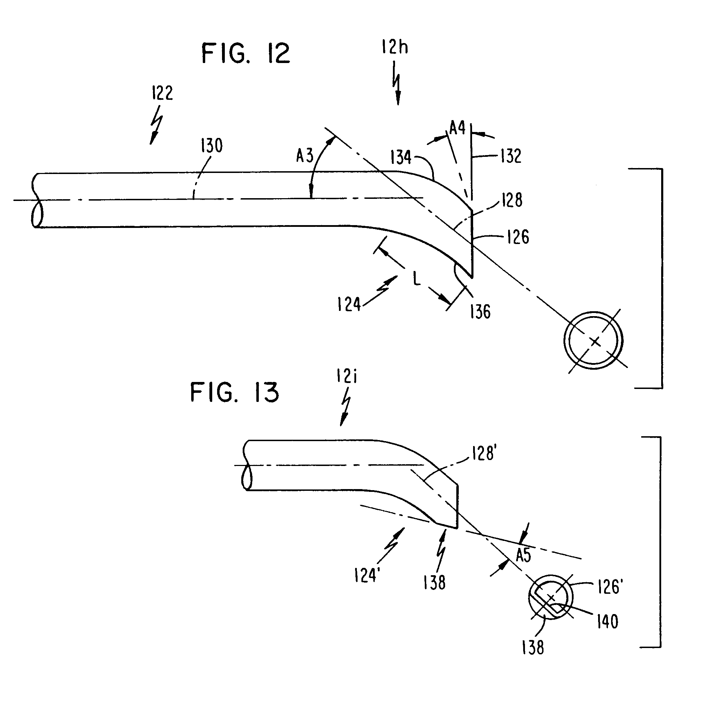

Reference is made to Figs. 9 and 10. These figures disclose other surgical tip assemblies 12f and 12g contemplated by the present invention for purposes of propagating cavitation bubbles which collapse in the fluid adjacent the tissue to be removed. In Fig. 9, the operative tip 12f is partially shown as including a generally smooth spherical tip portion 108 extending from an ultrasonically driven rod 110. The generally spherical tip portion 108 is formed so as to generate the micro-cavitation bubbles around the periphery thereof. The cavitation generated will be concentrated about the most distal end of the tip portion 108 and in the region whereat the rod 110 intersects the tip portion. Of course, it will be realized that the tip portion 108 can have a roughened surface or surface deformations which can serve to create the desired cavitation of the present invention. Suitable aspiration and irrigation sleeves (not shown) can be provided so as to surround the rod 110 much as in the manner shown in Fig. 8. The size of the tip portion 108 can vary depending on use thereof.

In Fig. 10, the operative tip assembly 12g is provided with a solid distal end portion 112 which has the surface 114 thereof roughened to provide the cavitation generating surface. The degree of roughing of the roughened surface 114 of the end portion 112 is effective for providing the desired collapsible micro-cavitation bubbles in the fluids surrounding the tissue. Accordingly, a wide variety of materials are contemplated so long as the surface roughness thereof is adequate for purposes of creating the micro-cavitation bubbles of the type noted and the materials are otherwise compatible for use in the type of surgery contemplated.

Reference is now made to Figs. 12 and 13 which illustrate further embodiments of the high-energy tip of the present invention. With reference to Fig. 12 firstly, another embodiment of a surgical tip assembly is designated by the reference numeral 12h and includes an elongated tubular tip member 122 and a bent cutting tip portion 124. It should be understood that the bent cutting tip portion is bent in the direction opposite to the tip assembly 12 depicted in Fig. 3. The bent cutting tip portion 124 has a cutting edge 126 thereon. The bent cutting tip portion 124 has a longitudinal axis 128 which forms an angle A3 to the longitudinal axis 130 of the elongated tip member 122, the angle A3 may range between 0° and 90° with a preferred range being between 0° and 45°. The length L of the bent cutting tip portion 124 can range between .04 in. to 0.10 in. The cutting edge 126 forms an angle A4 with the vertical axis 132 in a range between 0° and 30°. The vertical axis 132 is perpendicular to the longitudinal axis 130 of the elongated tubular tip member 122. In this configuration, the bent cutting tip portion 124 provides a first cavitation generating surface 134 during the forward stroke of the tip and a second cavitation generating surface 136 during the return stroke.

With reference to Fig. 13 and a further embodiment of the high-energy cutting tip depicted in Fig. 12, a high-energy cutting tip is generally designated by reference numeral 12i and includes a bent cutting tip portion 124′ having a blunt surface 138 thereon. The blunt surface 138 creates a flattened edge portion 140 on the cutting edge 126′. The blunt surface 138 forms an angle A5 with respect to the longitudinal axis 128′ of the bent cutting tip portion 124 which can range between 10° and 90° with an angle of about 30° depicted. The blunt surface 138 provides a further cavitation generating surface during use of the high-energy tip similar to the blunt surface depicted in Figures 3 and 3A.

Suitable aspiration and irrigation sleeves can be provided. It will be appreciated that the length and diameters of the foregoing tips can vary generally while being consistent with the surgical objectives desired and the formation of the noted cavitation bubbles.

During operation, the tips, as described above, emulsify a wide variety of tissues. The power level which is generally required to emulsify, on a given frequency system, was reduced up to about 50% compared to known tips. This, of course, leads to less strike or impact heating by the tip and the potential of corneal burning at the operative site is reduced. These tips have superior cutting and sculpting functions, when compared to a standard tip, and are less likely to cause clogging which results in occlusions during aspiration.

As noted earlier, the present invention contemplates selectively using the micro-cavitation bubbles for sculpting body tissue, such as the cornea. In such an embodiment, a preformed disk 100 (Fig. 11) is positioned at the end of the tip assembly 12f. The disk has a predetermined concave cavitation generating surface 102 which is placed in juxtaposed relationship to the cornea C. Ultrasonic vibrations will form the micro-cavitation bubbles in fluid bath 104 surrounding the cornea. As a result, there is selective removal of tissue. The concave surface 102 has the shape complementary to that desired for the sculpted cornea. The tissue will be destroyed in a zone determined by the shape of the surface 102. The bath of fluid 104 around the cornea C is necessary for the formation of the cavitation bubbles for achieving the desired tissue emulsification. It would be desired to have the patient horizontal so as to facilitate holding the bath around the cornea. Preferably, the surface 102 rides on the bath of fluid and not the cornea C during the sculpting operation.

The present invention, of course, also envisions utilizing such selective cavitation for removing a variety of tissue, such as tumors, cartilage or tissue obstructing arteries.

It will be seen from the previous embodiments that there are provided improved methods, apparatus and tip assemblies for achieving desired enhancement of emulsification at the operative site. Since certain changes may be made in the above described methods, apparatus and tip assemblies without departing from the scope of the invention herein involved, it is intended that all matter contained in the description and as shown in the accompanying drawings shall be interpreted as illustrative and not in a limiting sense.

**Claims**

1. A surgical tip assembly comprising:
   a surgical body having at least a cutting surface adjacent a distal tip portion thereof;
   said body having at least a cavitation generating surface.

2. The tip assembly of claim 1, wherein said cavitation generating surface is a blunt surface.

3. The tip assembly of claim 2, wherein said cutting edge is on a bent tip portion having a first predetermined angle relative to a longitudinal axis of said body and said blunt surface is at a second predetermined angle relative to the first predetermined angle.

4. The tip assembly of claim 2, wherein said cutting surface is a cutting edge which is engageable with the tissue to be removed and said blunt surface is adjacent to said cutting edge.

5. The tip assembly of claim 1, wherein said body portion has a plurality of micro-cavitation bubble generating surfaces.

6. The tip assembly of claim 1, wherein said distal tip portion has a double bend configuration with a first portion angularly bent relative to a first axis and a second distal portion generally parallel to the first axis and bent relative to the first bent portion so that it is offset transversely from the first axis, such that the direction of the stroke of said tip portion coupled with said bent portions produces collapsible micro-cavitation bubbles.

7. The tip assembly of claim 1, wherein said cavitation generating surface is generally spherical.

8. The tip assembly of claim 1, wherein said cavitation generating surface is roughened.

9. The tip assembly of claim 1, wherein said cavitation generating surface has a generally concave surface surrounded by said cutting edge which has a generally annular configuration.

10. The tip assembly of claim 1, wherein said body surface has an outer curvature and an inner curvature, each of which includes at least one of said cavitation generating surfaces.

11. The tip assembly of claim 1, further comprising at least a second collapsible micro-cavitation bubble generating surface adjacent the first generating surface.

12. The tip assembly of claim 1, wherein said cutting edge is on a bent tip portion having a first predetermined angle relative to a longitudinal axis of said body and said blunt surface is at a second predetermined angle relative to the first predetermined angle, said bent tip portion further including a second cavitation generating surface opposite the first cavitation generating surface.

13. The tip assembly of claim 12, further comprising a third cavitation generating surface adjacent the second cavitation generating surface.

14. The tip assembly of any of claims 1 to 13 further comprising:
    a surgical assembly;
    handpiece means housing transducer means operable for producing ultrasonic vibrational wave patterns;
    connecting means within said handpiece means for connecting said surgical tip assembly to said transducer means so that the vibrations are transferred to said tip assembly; and

aspirating means operable for allowing aspiration of tissue from a surgical site.

15. The apparatus of claim 14, wherein said surgical tip body includes an aspiration passage having an aspiration port being adjacent said cutting surface.

# FIG. 1

ULTRASONIC
TRANSDUCER

TO HANDPIECE

CONTROL
CONSOLE

TISSUE
COLLECTION
VESSEL

FIG. 2

FIG. 3A

FIG. 3

EP 0 482 847 A1

FIG. 4

FIG. 6

FIG. 6A

FIG. 7

FIG. 8

FIG. 5

ASPIRATION    IRRIGATION

FIG. 9

FIG. II

FIG. 10

FIG. 12

FIG. 13

EP 0 482 847 A1

**European Patent Office**

## EUROPEAN SEARCH REPORT

Application Number

EP 91 30 9671

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-0 238 667 (SUMITOMO BAKELITE COMPANY LIMITED) <br> * page 4, line 14 - page 5, line 3 * <br> * page 7, line 6 - page 9, line 4 * <br> * abstract; claims 1,2; figures 1-4,6 * <br> --- | 1-5,11, 14,15 | A61F9/00 |
| X | GB-A-2 118 045 (GORKOVSKY GOSUDARSTVENNY MEDITSINSKY INSTITUT IMENI S.M. KIROVA) <br> * page 2, line 25 - line 37; figures 1-3 * <br> --- | 1,2,4,9, 14,15 | |
| X | US-A-4 959 049 (SMIRMAUL) <br><br> * column 1, line 48 - column 2, line 5 * <br> * abstract; claims 1,2; figures 1-5 * <br> --- | 1-4,14, 15 | |
| X | US-A-4 689 040 (THOMPSON) <br> * abstract; figures * <br> --- | 1,14,15 | |
| A,D | US-A-4 223 676 (WUCHIN) <br> * figure 1 * <br><br> ----- | 14,15 | TECHNICAL FIELDS SEARCHED (Int. Cl.5 ) <br><br> A61F <br> A61B |

The present search report has been drawn up for all claims

| Place of search | Date of completion of the search | Examiner |
|---|---|---|
| THE HAGUE | 10 FEBRUARY 1992 | GIMENEZ BURGOS R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)